# EUROPEAN PATENT SPECIFICATION

(11) **EP 3 536 289 B1**
(45) Date of publication and mention of the grant of the patent: **21.04.2021**
(21) Application number: 19160038.6
(22) Date of filing: 10.10.2011
(51) Int. Cl.: A61F 9/008, A61F 9/009

(54) **SYSTEM AND INTERFACE DEVICES FOR EYE SURGERY**
SYSTEM UND SCHNITTSTELLENVORRICHTUNGEN FÜR DIE AUGENCHIRURGIE
SYSTÈME ET DISPOSITIFS D'INTERFACE DE CHIRURGIE OCULAIRE

(43) Date of publication of application: 11.09.2019
(62) Divisional of application: 11770701.8
(73) Proprietor: WaveLight GmbH, 91058 Erlangen (DE)
(72) Inventor: GORSCHBOTH, Claudia, 90411 Nürnberg (DE); VOGLER, Klaus, 90542 Eckental/Eschenau (DE); DONITZKY, Christof, 90542 Eckental/Eschenau (DE)
(74) Representative: Frenkel, Matthias Alexander

(56) References cited:
- WO-A1-94/09849
- WO-A1-98/19642
- WO-A1-2008/064771
- WO-A1-2011/069516
- US-A- 3 467 099
- US-A- 4 506 962
- US-A- 5 226 903
- US-A- 5 549 632
- US-A- 6 019 472
- US-A1- 2009 069 794
- US-A1- 2010 324 543

## Description

The invention relates generally to laser-surgical treatment of the human eye. In particular, the invention relates to the application of various forms of treatment of the human eye with the aid of one and the same eye-surgical laser apparatus.

The use of focused pulsed laser radiation for the purpose of generating incisions in the corneal tissue or in other tissue parts of the human eye has been the subject of intense research in human ophthalmology for some time. Instruments are also already on the market that provide a function of incision generation with laser radiation of such a type. Ordinarily in this connection, ultra-short-pulse laser radiation with pulse durations within the femtosecond range comes into operation. However, the invention is not restricted thereto; to the extent that generation of an incision in corneal eye tissue is possible also with shorter or longer pulse durations, these are likewise to be encompassed by the invention; for example, pulse durations within the attosecond range or within the one-digit, two-digit or three-digit picosecond range. The physical effect that is utilised in the course of generating an incision by means of pulsed laser radiation is so-called laser-induced optical breakthrough, which results in a so-called photodisruption, the magnitude of which is limited roughly to the extent of the radiation focus at the waist point of the radiation. As a result of juxtaposing a large number of such photodisruptions, diverse and comparatively complex incision figures can be generated in the eye tissue.

An exemplary application of the generation of an incision by means of pulsed laser radiation is so-called LASIK (laser in-situ keratomileusis). In this surgical procedure - which is generally to be classified as refractive surgery, that is to say, surgery aimed at the elimination or at least improvement of defective imaging properties of the eye - firstly the human cornea is cut open horizontally (from the point of view of the reclining patient), whereby a small cover (ordinarily called a flap in the specialist field) arises which can be folded aside. After the flap has been folded away, in the stroma of the cornea that has been exposed in this way a so-called ablation is effected by means of laser radiation (for example, excimer radiation with a wavelength of 193 nm), i.e. stromal tissue is respected in accordance with a suitable ablation profile computed beforehand for the patient. After this, the small cover (flap) is folded back, at which point the healing process proceeds comparatively painlessly and quickly. After this intervention the cornea has different imaging properties, in which connection a largely complete elimination of the previous visual disorder is achieved in the best case.

As an alternative to the prior 'classical' procedure (mechanical microkeratome), the cutting of the flap can also be realised using laser technology. The existing conceptions for this frequently provide for an applanation (levelling) of the anterior surface of the cornea by abutment of a planar abutment face of a contact element that is transparent to the laser radiation, the flap then being generated by a bed incision situated at constant depth and by a lateral incision extending from the bed incision as far as the surface of the cornea. The levelling of the cornea permits the bed incision to be executed as a two-dimensional incision, for which solely a control of the location of the radiation focus in a plane perpendicular to the direction of propagation of the radiation (designated in conventional notation as the x-y plane) is required, without undertaking a control of the location of the radiation focus in the direction of propagation of the laser radiation (this direction is designated, according to conventional notation, as the z-direction). If a control of the location of the radiation focus in the z-direction is to be effected, this can be done, for example, with the aid of a liquid lens, as described, for example, in EP 1 837 696 from the present applicant. In this respect, reference is made to the aforementioned patent application, the content of which is hereby incorporated into this application. In general, the control of the location of the radiation focus is described in patent applications EP 2 111 831 and WO 2010/142311 from the present applicant, the content of which is hereby incorporated into this application.

Another form of operation in which incisions are generated in the cornea by means of pulsed laser radiation is laser-assisted corneal lenticle extraction. In this case, in the stroma of the cornea a tissue volume - which, for example, has the shape of a small disc - is cut free which can be extracted from the eye through an auxiliary incision. Depending upon the indication (e.g. myopia, hyperopia), the lenticle to be removed may have varying shapes. For the purpose of cutting the lenticle free, the procedure hitherto has frequently been such that firstly a lower incision surface bounding the underside of the lenticle (posterior side of the lenticle) and subsequently an upper incision surface bounding the upper side of the lenticle (anterior side of the lenticle) are generated in the cornea, both incision surfaces frequently being three-dimensional and each requiring a z-control of the radiation focus.

For the purpose of x-y- adjustment of the radiation focus in the cornea, sufficiently fast scanners are available which, for example, operate with galvanometrically controlled scanner mirrors. On the other hand, available z-scanners - that is to say, scanners that enable a focus displacement in the z-direction - are frequently comparatively slow in comparison with galvanometric mirror scanners. In addition, only a limited z-range can be covered with the available z-scanners.

In contrast to the refractive eye surgery described previously on the basis of LASIK and also on the basis of corneal lenticle extraction, in which incisions are generated in the cornea, in the case of cataract surgery incisions are implemented in the lens of the human eye. However, solely by virtue of the z-displacement of the laser focus of the laser beam of a laser apparatus being used for the refractive eye surgery with the aid of the z-scanner the focus cannot reach so far into the eye with sufficiently good quality that an incising in the lens (i.e. deeper within the eye) is possible with the same quality as in the cornea

US 2010/324543 A1 discloses an integrated ophthalmic procedure where a surgical laser has a patient interface, including a contact lens which can be a flat applanation plate or a curved lens. In integrated procedures, the contact lens can be changed from a contact lens, optimized for cataract procedures to another contact lens, optimized for glaucoma procedures.

It is accordingly an object of the present invention to provide a laser system with an eye-surgical laser apparatus, the laser apparatus itself, and a set of interface devices for use in the eye-surgical laser apparatus, by means of which various types of treatment can be carried out with the same eye-surgical laser apparatus.

This object is achieved by the subject-matter of the independent claim. Advantageous embodiments arise out of the dependent claims.

Examples, aspects and embodiments described in the following disclosure, in particular methods, which do not fall under the scope of the claims do not constitute part of the invention and are presented for illustration purposes only.

In the following, the terms 'interface device', 'interface unit', 'patient interface', 'patient adapter' and 'eye interface' will be used alternately but are to be understood as being synonymous.

According to a first aspect of the present disclosure, the laser system according to the invention for eye surgery comprises an eye-surgical laser apparatus and a set of interface devices (patient/eye interfaces). The eye-surgical laser apparatus includes optical components for providing pulsed focused laser radiation with radiation properties matched to the generation of photodisruptions in human eye tissue and a control unit for positional control of the radiation focus of the laser radiation. The control unit is designed for executing various control programs that represent various types of incision figure. Each of the interface devices of the set includes a contact body that is transparent to the laser radiation, with an abutment face for abutment against an eye to be treated, and also a coupling portion for detachable coupling of the interface device (patient interface) onto a counter-coupling portion of the laser apparatus, the interface devices of the set differing by virtue of a differing optical effect on the laser radiation provided in the laser apparatus, for example on the laser radiation emerging from the laser apparatus.

It is possible that at least a subset of the interface devices differ by virtue of a differing influence on the location of the radiation focus relative to the abutment face.

The differing optical effect may, for example, consist in the fact that, depending upon the coupling of one of the interface devices onto the counter-coupling portion, the focal point of the laser radiation relative to the abutment face in the case of one and the same laser apparatus comes to be situated at a different position in the eye (i.e. at a different focus location). For example, depending upon the coupled interface device the focus location (the position of the focal point) may come to be situated in the cornea of the eye, in the lens of the eye or at a different point on or in the eye, for example in the iridocorneal angle of the eye. It is, for example, conceivable that the focus location (i.e. how deep the focal point is situated in the eye in the z-direction with respect to the abutment face) in the case of coupling of a first interface device is between 250 µm and 350 µm, in particular between 280 µm and 320 µm and preferentially at 300 µm. Such an interface device would be suitable for operational application for the purpose of machining the cornea with the aid of the eye-surgical laser apparatus. Similarly, it is conceivable that the focus location in the case of coupling of a second interface device lies, for example, between 4 mm and 6 mm, in particular between 4.5 mm and 5.5 mm and preferentially 5 mm, below a contact lens of the interface device. Such an interface device would be suitable for operational application for the purpose of machining the lens with the aid of the eye-surgical laser apparatus.

The differing optical effect may further consist in the fact that, depending on the coupled interface device, a different range of adjustment in the z-direction (i.e. a different range of depth of focus) is possible or is set with one and the same laser apparatus. For example, depending upon the coupled interface device the range of depth of focus (i.e. the range of adjustment of the focal point in the z-direction) may have been matched or may be matched to the machining of the cornea of the eye, of the lens of the eye or of another point on or in the eye. It is, for example, conceivable that the range of depth of focus (i.e. how far the focal point can be adjusted with the aid of a z-scanner in the z-direction of one and the same laser apparatus) in the case of coupling of a first interface device is between 1 mm and 1.4 mm, in particular between 1.1 mm and 1.3 mm and preferentially at 1.2 mm. Such an interface device would be suitable for operational application for the purpose of machining the cornea with the aid of the eye-surgical laser apparatus. Similarly, it is conceivable that the range of depth of focus in the case of coupling of a second interface device lies between 8 mm and 16 mm, in particular between 10 mm and 14 mm and preferentially at 12 mm. Such an interface device would be suitable for operational application for the purpose of machining the lens with the aid of the eye-surgical laser apparatus. The value of the depth of focus that is required for the respective application may be set, for example, by a z-scanner and the patient interface.

The differing optical effect may further consist in the fact that, depending on the coupled interface device, a differing spot diameter of the focal point is obtained with one and the same laser apparatus. For example, depending upon the coupled interface device, the spot diameter of the focal point may have been matched to the machining of the cornea of the eye, of the lens of the eye or of another point on or in the eye. It is, for example, conceivable that the spot diameter in the case of coupling of a first interface device lies between 1 µm and 6 µm, in particular between 2 µm and 5 µm and preferentially between 3 µm and 5 µm. Such an interface device would be suitable for operational application for the purpose of machining the cornea with the aid of the eye-surgical laser apparatus. Similarly, it is conceivable that the spot diameter in the case of coupling of a second interface device lies between 3 µm and 14 µm, in particular between 4 µm and 12 µm and preferentially between 5 µm and 10 µm. Such an interface device would be suitable for operational application for the purpose of machining the lens with the aid of the eye-surgical laser apparatus.

The differing optical effect may further consist in the fact that, depending on the coupled interface device, a differing scan-field diameter (i.e. a differing diameter of the region that is capable of being irradiated by the laser beam in the x-y direction/plane) is obtained with one and the same laser apparatus. For example, depending upon the coupled interface device, the scan-field diameter may have been matched to the machining of the cornea of the eye, of the lens of the eye or of another point on or in the eye. It is, for example, conceivable that the scan-field diameter in the case of coupling of a first interface device lies between 9 mm and 15 mm, in particular between 11 mm and 13 mm, and preferentially amounts to 12 mm. Such an interface device would be suitable for operational application for the purpose of machining the cornea with the aid of the eye-surgical laser apparatus. Similarly, it is conceivable that the scan-field diameter in the case of coupling of a second interface device lies between 5 mm and 9 mm, in particular between 6 mm and 8 mm, and preferentially amounts to 7 mm. Such an interface device would be suitable for operational application for the purpose of machining the lens with the aid of the eye-surgical laser apparatus.

The differing optical effect on the laser radiation provided in the laser apparatus preferably has the result that a small and at least almost equally large (uniform) focus is present in all machining regions within the eye. In particular, this results in a good, adapted focusing, in a low pulse energy of the laser radiation, and/or in a slight burdening of the patient.

Furthermore, at least a subset of the interface devices may differ by virtue of a differing shape and/or location of at least one optical boundary surface. The optical boundary surface may be, for example, a face of a contact lens that is present in the corresponding interface device, which usually serves for abutment of the eye. It is also possible that the optical boundary surface is constituted by a face of an optical ancillary element that is present in the interface device in addition to the contact lens. Accordingly, it is also conceivable that at least a subset of the interface devices differ by virtue of a differing number of optical elements. These optical elements may, for example, include the contact lens for abutment against the eye or the optical ancillary element, for example a lens (refractive optical element) or a diffractive optical element.

At least one of the interface devices may include an applanation cone that is designed to be coupled onto the eye and onto focusing optics of the laser apparatus. However, several, a large number of or all of the interface devices may include an applanation cone of such a type.

The laser apparatus may further include an adaptive optical element that is arranged upstream of focusing optics of the laser apparatus in the direction of propagation of the laser radiation. The adaptive optical element may include an adaptive mirror or a light-transmitting adaptive system. The adaptive optical element may in this case provide for the compensation of a possibly increased wavefront aberration. Such an increase may occur, for example, if a laser system is used for differing applications. In particular, an enlarged range of depth of focus that is required for this may necessitate a correction in the course of incising in the lens.

At least one of the interface devices may include a coding/code that enables the laser apparatus to execute, depending on the coding/code, the control program in the control unit. For example, the laser apparatus may recognise the coding/code and call an associated control program (assigned to the coding/code) in the control unit, preferentially automatically.

According to a second aspect of the present disclosure, a set of interface devices is made available for use in the eye-surgical laser apparatus, for example in each instance an interface device (patient interface) for the respective intraocular application. Each of the interface devices includes a contact body that is transparent to the laser radiation of the laser apparatus, with an abutment face for abutment against an eye to be treated, and also a coupling portion for detachable coupling of the interface device onto a counter-coupling portion of the laser apparatus. The interface devices differ: (i) by virtue of a differing influence on the location of a radiation focus of the laser radiation relative to the abutment face and/or (ii) by virtue of a differing shape and/or location of at least one optical boundary surface and/or (iii) by virtue of a differing number of optical elements, resulting in a differing optical effect on the laser radiation made available in the laser apparatus (e.g. resulting in a differing focusing of the beam, deflection of the beam and/or splitting of the beam). In particular, by virtue of the interface devices a differing treatment region (e.g. range of depth of focus) in the x-y- direction and/or in the z-direction can be covered, depending upon which interface device is connected to the laser apparatus.

At least one or a subset of the interface devices may include a planar contact lens. In the case of such a planar contact lens a face that is suitable for abutment against the eye takes the form of a planar abutment face and the face situated opposite the abutment face (the face facing away from the eye) is designed to be plane-parallel to the abutment face. At least one of the interface devices may include an optical ancillary element. For example, the optical ancillary element may be present in the interface device or in one of the interface devices with a planar contact lens. The optical ancillary element may, for example, have been arranged in the interface device in such a manner that a face facing away from the contact lens is shaped in convex or planar manner and a face facing towards the contact lens is concavely shaped. However, other designs of the optical ancillary element are also conceivable. Irrespective of the precise shape of the optical ancillary element, the face facing towards the contact lens and/or the face of the optical ancillary element facing away from the contact lens may have been formed as an optical freeform surface.

At least one of the interface devices may also include a concavo-concave contact lens. In the case of a concavo-concave contact lens of such a type a concave abutment face is provided for abutment against the eye, the face situated opposite the abutment face being concavely shaped. Additionally or alternatively, at least one of the interface devices may include a concavo-convex or concavo-planar contact lens. In the case of a concavo-convex contact lens, a concave abutment face is provided for abutment against the eye and the face situated opposite the abutment face is convexly shaped. In the case of a concavo-planar contact lens, a concave abutment face is provided for abutment against the eye and the face situated opposite the abutment face is shaped in planar manner. Irrespective of the precise configuration of the contact lens, the abutment face and/or the face situated opposite the abutment face may take the form of an optical freeform surface with refractive or diffractive effect.

According to a third aspect of the present disclosure, use is made of a set of interface devices, the use including the variable operational application of, in each instance, one of the interface devices in an eye-surgical laser apparatus. The laser apparatus includes optical components for making available pulsed focused laser radiation with radiation properties matched to the generation of photodisruptions in human eye tissue and a control unit for positional control of the radiation focus of the laser radiation. The control unit is further designed for executing various control programs that represent various types of incision figure, each of the interface devices including a contact body that is transparent to the laser radiation, with an abutment face for abutment against an eye to be treated, and also a coupling portion for detachable coupling of the interface device onto a counter-coupling portion of the laser apparatus. The interface devices of the set differ by virtue of a differing optical effect on the laser radiation provided in the laser apparatus, and the use includes the operational application of various interface devices of the set, depending on the control program to be executed in the given case.

At least a subset of the interface devices may differ by virtue of a differing influence on the location of the radiation focus relative to the abutment face. Furthermore, at least a subset of the interface devices may differ by virtue of a differing shape and/or a differing location of at least one optical boundary surface. It is also conceivable that at least a subset of the interface devices differ by virtue of a differing number of optical elements.

In the case of an exchange of the interface device the focusing setting of focusing optics of the laser apparatus may remain unchanged. Consequently a differing optical effect in the laser apparatus can be achieved with one and the same laser apparatus by virtue of the fact that the interface devices for the respective application are exchanged and the focusing settings remain unchanged.

In the case of an exchange of the interface device the control unit can control the laser apparatus in such a manner that an adaptive optical element or a light-transmitting adaptive system is introduced into the beam path of the laser radiation. For this purpose a corresponding coding/code on the interface devices may be present, on the basis of which an identification of the interface device takes place. The associated adaptive element or system (assigned to the coding/code) can then be introduced, for example automatically, into the beam path in accordance with the identification. The adaptive optical element or the light-transmitting adaptive system can also be introduced upstream of focusing optics of the laser radiation in the direction of propagation of the laser radiation.

According to a fourth aspect of the present disclosure, a method for laser-surgical eye treatment is made available wherein pulsed focused laser radiation with radiation properties matched to the generation of photodisruptions in human eye tissue is provided by means of a laser apparatus and the position of the radiation focus of the laser radiation is controlled by means of a control unit, wherein in the case of a first treatment-type a sequence of at least one control program that represents a first type of incision figure is executed by means of the control unit, whereby a first interface device matched to the first treatment-type is placed over a contact body that is transparent to the laser radiation with an abutment face against an eye to be treated, and via a coupling portion is detachably coupled onto a counter-coupling portion of the laser apparatus, wherein in the case of a second treatment-type a sequence of the at least one control program that represents a second type of incision figure, different from the first type of incision figure, is executed by means of the control unit, whereby a second interface device matched to the second treatment-type is placed over a contact body that is transparent to the laser radiation, with an abutment face against an eye to be treated, and via a coupling portion is detachably coupled onto a counter-coupling portion of the laser apparatus. The aforementioned coding/code of the interface devices may serve to ensure that the associated control program is, for example, automatically recognised, set and executed.

The first treatment-type may include a treatment of the cornea of the eye by means of the laser radiation. The second treatment-type may include a treatment of the lens of the eye by means of the laser radiation.

In an alternative embodiment it is conceivable that the second treatment-type includes a treatment of the iris, of the retina, of the vitreous body or of regions of the iridocorneal angle (e.g. for the purpose of treating glaucoma) of the eye by means of the laser radiation.

In the case of a third treatment-type, a sequence of the at least one control program that represents a third type of incision figure, different from the first and/or second type of incision figure, may be executed by means of the control unit, whereby a third interface device matched to the third treatment-type may be placed over a contact body that is transparent to the laser radiation, with an abutment face against an eye to be treated, and via a coupling portion may be detachably coupled onto a counter-coupling portion of the laser apparatus and the third treatment-type may include a treatment of the iris of the eye by means of the laser radiation.

In the case of a fourth treatment-type, a sequence of the at least one control program that represents a fourth type of incision figure, different from the first, second and/or third type of incision figure, may be executed by means of the control unit, whereby a fourth interface device matched to the fourth treatment-type may be placed over a contact body that is transparent to the laser radiation, with an abutment face against an eye to be treated, and via a coupling portion may be detachably coupled onto a counter-coupling portion of the laser apparatus and the fourth treatment-type may include a glaucoma treatment in the iridocorneal angle of the eye by means of the laser radiation.

In the case of a fifth treatment-type, a sequence of the at least one control program that represents a fifth type of incision figure, different from the first, second, third and/or fourth type of incision figure, may be executed by means of the control unit, whereby a fifth interface device matched to the fifth treatment-type may be placed over a contact body that is transparent to the laser radiation, with an abutment face against an eye to be treated, and via a coupling portion may be detachably coupled onto a counter-coupling portion of the laser apparatus and the fifth treatment-type may include a treatment of the vitreous body of the eye by means of the laser radiation.

In the case of a sixth treatment-type, a sequence of the at least one control program that represents a sixth type of incision figure, different from the first, second, third, fourth and/or fifth type of incision figure, may be executed by means of the control unit, whereby a sixth interface device matched to the sixth treatment-type may be placed over a contact body that is transparent to the laser radiation, with an abutment face against an eye to be treated, and via a coupling portion may be detachably coupled onto a counter-coupling portion of the laser apparatus and the sixth treatment-type may include a treatment of the retina of the eye by means of the laser radiation.

The disclosure will be elucidated further in the following on the basis of the appended drawings, which are schematic throughout. Shown are:
- Figure 1: a schematic block representation of elements of a laser device for eye-surgical treatments according to one embodiment;
- Figure 2a: a schematic representation of a beam path of a laser beam for machining the cornea of a human eye;
- Figure 2b: a schematic representation of a beam path of a laser beam for machining the lens of a human eye;
- Figure 2c: a schematic representation of a beam path of a laser beam for machining the iris of a human eye;
- Figure 2d: a schematic representation of a beam path of a laser beam for machining the iridocorneal angle of a human eye;
- Figure 2e: a schematic representation of a beam path of a laser beam for machining the vitreous body of a human eye;
- Figure 2f: a schematic representation of a beam path of a laser beam for machining the retina of a human eye;
- Figure 3: a further schematic representation of the beam path of the laser beam for machining the lens from Figure 2b;
- Figure 4a: a schematic representation of a first interface device for use in the laser device according to Figure 1;
- Figure 4b: a schematic representation of a second interface device for use in the laser device according to Figure 1;
- Figure 4c: a schematic representation of a third interface device for use in the laser device according to Figure 1;
- Figure 4d: a schematic representation of a fourth interface device for use in the laser device according to Figure 1;
- Figure 4e: a schematic representation of a fifth interface device for use in the laser device according to Figure 1; and
- Figure 4f: a schematic representation of a sixth interface device for use in the laser device according to Figure 1.

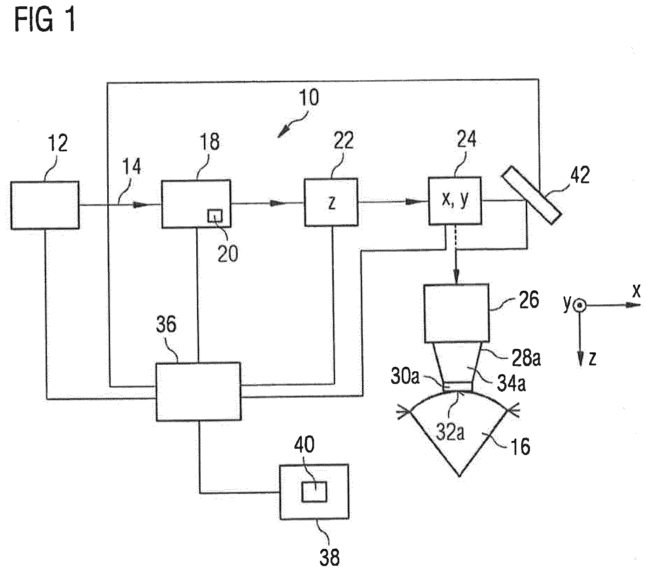

The laser device shown in Fig. 1 - denoted generally therein by 10 - comprises a laser source 12 which makes available a pulsed laser beam 14, in the case of which the pulse duration of the radiation pulses is suitable for use of the laser beam 14 for the purpose of generating incisions in the corneal tissue of an eye 16 of a patient to be treated. For example, the pulse duration of the radiation pulses of the laser beam 14 lies within the nanosecond, picosecond, femtosecond or attosecond range. The laser beam 14 made available by the laser source 12 has a pulse repetition rate such as is desired for the application in question, i.e. the repetition rate of the radiation pulses emitted from the laser device 10 and directed onto the eye 16 corresponds to the repetition rate of the radiation pulses that are available at the output of the laser source 12, unless, in a manner depending on the machining profile predetermined for the eye 16, a partial number of the radiation pulses emitted from the laser source 12 are blanked by means of an optical switch 18 arranged in the radiation path of the laser beam 14. Such blanked radiation pulses accordingly do not reach the eye 16.

In a manner not shown in any detail but known as such, the laser source 12 may include, for example, a laser oscillator (e.g. solid-state laser oscillator), a preamplifier, which increases the pulse power of the laser pulses emitted from the oscillator and simultaneously temporally stretches them, a subsequent pulse-picker, which selects individual laser pulses from the pre-amplified laser pulses of the oscillator, in order in this way to lower the repetition rate to a desired degree, a power amplifier, which amplifies the selected, still temporally stretched, pulses to the pulse energy needed for the application, and a pulse compressor, which temporally compresses the pulses output from the power amplifier to the pulse duration desired for the application.

The optical switch 18, which may also be designated as a pulse modulator, may, for example, take the form of an acousto-optical modulator or an electro-optical modulator. Generally, the optical switch 18 may contain arbitrary optically active elements that enable a rapid blanking of individual laser pulses. The optical switch 18 may, for example, contain a beam trap, indicated schematically at 20, which serves to absorb radiation pulses to be blanked, which are not to reach the eye 16. The optical switch 18 can deflect such radiation pulses to be blanked from the normal beam path of the radiation pulses of the laser beam 14 and direct them onto the beam trap 20.

In the beam path of the laser beam 14 further optical components are arranged which, in the exemplary case shown, include a z-scanner 22, an x-y scanner 24 and also a focusing objective 26. The focusing objective 26 serves for focusing the laser beam 14 onto a desired machining location on or in the eye 16, in particular in the cornea of the same. The z-scanner 22 serves for longitudinal control of the location of the focal point of the laser beam 14; the x-y scanner 24 serves, on the other hand, for transverse control of the location of the focal point. 'Longitudinal' relates in this connection to the direction of beam propagation; this is designated in conventional notation as the z-direction. 'Transverse', on the other hand, designates a direction transverse to the direction of propagation of the laser beam 14; according to conventional notation the transverse plane is designated as the x-y plane. A coordinate frame that represents the x-y-z directions in the region of the eye 16 has been drawn in Fig. 1 for the purpose of illustration.

For the purpose of transverse deflection of the laser beam 14, the x-y scanner 24 may, for example, include a pair of galvanometrically actuated scanner mirrors that are capable of tilting about mutually perpendicular axes. On the other hand, the z-scanner 22 may, for example, contain a longitudinally adjustable lens or a lens of variable refractive power or a deformable mirror, with which the divergence of the laser beam 14 and consequently the z-position of the beam focus can be influenced. For example, such an adjustable lens or mirror may be contained in a beam expander which is not represented in any detail and which expands the laser beam 14 emitted from the laser source 12. The beam expander may, for example, be configured as a Galilean telescope.

The focusing objective 26 is preferably an f-theta objective and is preferentially detachably coupled on its beam-exit side with a patient adapter 28a which constitutes an abutment interface for the cornea of the eye 16. For this purpose the patient adapter 28a includes a contact element 30a that is transparent to the laser radiation and that on its underside facing towards the eye includes an abutment face 32a for the cornea of the eye 16. In the exemplary case shown, the abutment face 32a is realised as a plane surface and serves for levelling the cornea, by the contact element 30a being pressed against the eye 16 with appropriate pressure or by the cornea being aspirated onto the abutment face 32a by underpressure. The contact element 30a, which in the case of plane-parallel design is ordinarily designated as the applanation plate, is fitted to the narrower end of a conically widening carrier sleeve 34a. The connection between the contact element 30a and the carrier sleeve 34a may be permanent, for example by virtue of adhesion bonding, or it may be detachable, for instance by virtue of a screw coupling. It is conceivable, furthermore, to use an optical injection-moulded part with the functions of the carrier sleeve_34a and of the contact element 30a . In a manner not represented in any detail, the carrier sleeve 34a has at its wider sleeve end, which in the drawing is the upper end, suitable coupling structures for coupling onto the focusing objective 26.

It will be understood that the order of the optical switch 18, the z-scanner 22, the x-y scanner 24 and the focusing objective 26 does not have to be as represented in Fig. 1. For example, the optical switch 18 may readily have been arranged in the beam path downstream of the z-scanner 22. To this extent, the order of these components shown in Fig. 1 is in no way to be understood as restrictive.

The laser source 12, the optical switch 18 and also the two scanners 22, 24 (which, if desired, may also have been combined within a single structural unit) are controlled by a control computer 36 which operates in accordance with a control program 40 stored in a memory 38. The control program 40 contains instructions (program code) that bring about, upon execution by the control computer 36, such a control of the location of the beam focus of the laser beam 14 that in the cornea, in the lens or at another location of the eye 16 bearing against the contact element 30a an incision figure arises that, for example in the course of a machining of the cornea, completely severs from the surrounding corneal tissue a corneal tissue volume to be removed within the scope of a corneal lenticle extraction or a corneal keratoplasty. If desired, this incision figure may additionally bring about a segmentation of this tissue volume into a plurality of volume segments individually separated from one another.

Furthermore, an adaptive optical element or adaptive optical system, taking the form, in exemplary manner, of a mirror 42, may be capable of being introduced into the radiation path of the laser beam 14 upstream of the focusing objective 26. This mirror 42 may have been designed as a deformable mirror. Furthermore, instead of the mirror 42 another adaptive optical element or a light-transmitting adaptive system may have been provided. The mirror 42 is preferentially introduced into the radiation path of the laser beam 14 if a machining of the lens of the eye 16 is to be undertaken in order to lessen (compensate) wavefront aberrations. In the course of a machining of the cornea of the eye 16 the mirror 42 may be located in a null position (inactive position) in which the radiation path that is dashed in Fig. 1 is used, without the laser beam 14 passing through the mirror 42 (without the mirror 42 influencing the laser beam 14). The control of the radiation path (e.g. whether or not the mirror 42 is introduced into the radiation path) can be implemented by the control computer 36. In an alternative embodiment the mirror remains in the beam path, so that a drive, depending on the application, is effected via an activation of the application.

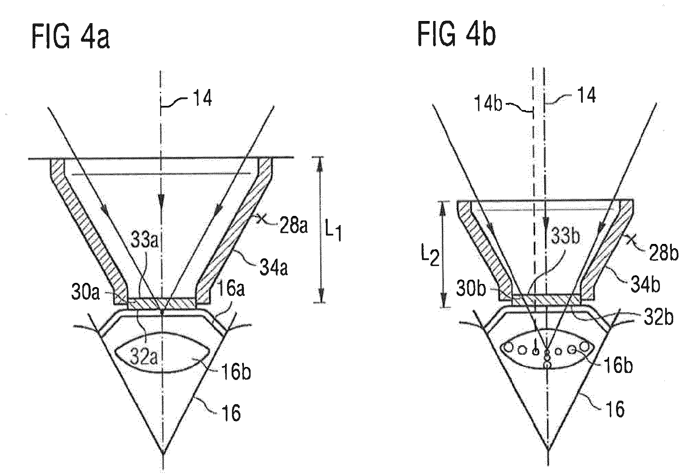

In the case of the laser device 10 shown in Figure 1 the patient adapter (interface unit) 28a is coupled in exemplary manner with the focusing objective 26. Accordingly, the eye 16 in Figure 1 is bearing against the planar abutment face 32a of the contact element 30a pertaining to the patient adapter 28a. This patient adapter 28a is shown in more detail in Figure 4a. Patient adapters 28b to 28e represented in Figures 4b to 4e form, jointly with patient adapter 28a from Figure 4a, a set of patient adapters, all of which are preferentially capable of being coupled with the same focusing objective 26. The further patient adapters 28b to 28e will be described more precisely with reference to Figures 4b to 4e. Firstly, however, it will be demonstrated generally which influence differing patient adapters have on the optical effect of the laser device 10.

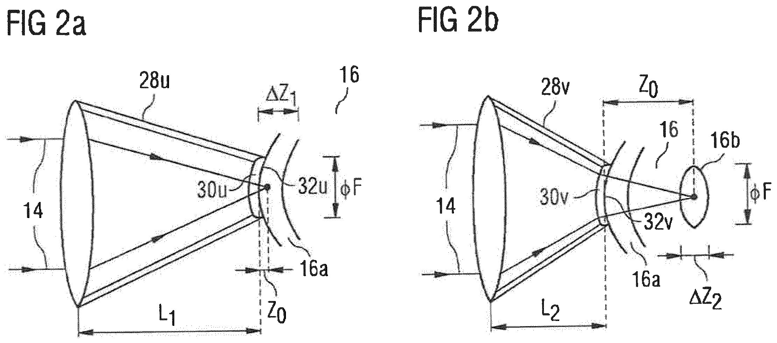

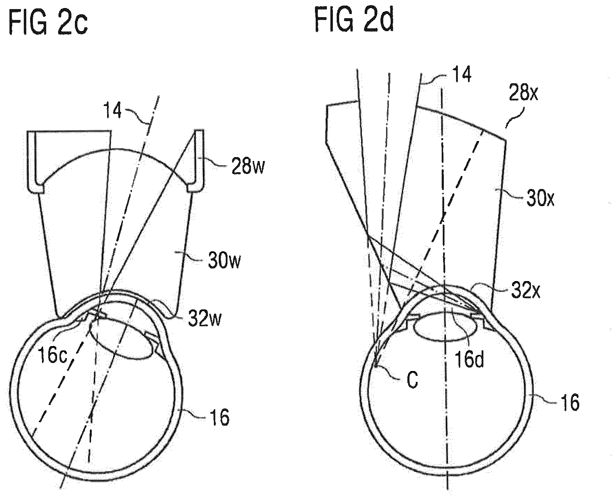

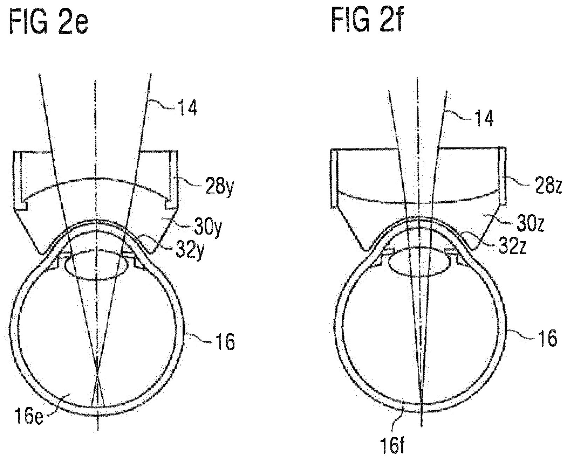

In Figures 2a to 2f six different types of patient adapter 28u, 28v, 28w, 28x, 28y, 28z are shown. Patient adapter 28u includes a contact lens 30u with an abutment face 32u for abutment against the eye 16 and enables a machining of the cornea 16a of the eye 16 with the aid of the laser beam 14. On the other hand, patient adapter 28v includes a contact lens 30v with an abutment face 32v for abutment against the eye 16 and enables a machining of the lens 16b of the eye 16 with unchanged setting of the laser device 10. Accordingly, with the same laser device 10 (and, for example, with identical setting of the same) a change of the optical effect of the laser device 10 can be obtained. Furthermore, patient adapter 28w enables a machining of the iris 16c of the eye 16 with the aid of the laser beam 14; patient adapter 28x enables a machining of the iridocorneal angle 16d of the eye 16 with the aid of the laser beam 14; patient adapter 28y enables a machining of the vitreous body 16e of the eye 16 with the aid of the laser beam 14; and patient adapter 28z enables a machining of the retina 16f of the eye 16 with the aid of the laser beam 14. Patient adapter 28w includes a contact lens 30w with an abutment face 32w for abutment against the eye 16; patient adapter 28x includes a contact lens 30x with an abutment face 32x for abutment against the eye 16; patient adapter 28y includes a contact lens 30y with an abutment face 32y for abutment against the eye 16; and patient adapter 28z includes a contact lens 30z with an abutment face 32z for abutment against the eye 16.

The optical effect of the laser device 10 in the case where use is made of patient adapter 28u is distinguished by the fact that the laser beam 14 is focussed in the cornea 16a. This means, inter alia, that the focal point of the laser beam 14 is situated in the cornea. For the machining of the cornea 16a, for a typical eye it is advantageous that the focus location z₀ (i.e. the spacing of the focal point from the abutment face 32u of the patient adapter 28u for abutment of the eye 16 for a defined state of the z-scanner 22) may attain a value of about 110 µm. In addition, for the machining of the cornea usually a variable setting of the depth of focus of Δz = 0 ... 1200 µm is required - that is to say, a range of adjustment of the focal point of about 1.2 mm. Furthermore, normally a spot diameter of the focal point of around 3-5 µm and a scan-field diameter Φ_{F} of around 12 mm are required. These properties are satisfied, for example, by patient adapter 28u.

If the settings of the laser device 10 are retained and only patient adapter 28u is replaced by patient adapter 28v, the focal point of the laser beam 14 does not lie in the cornea 16a, but in the lens 16b of the eye 16 (the mean focus location z₀ assumes, for example, a value of 5 mm). This is obtained by virtue of a shorter length L₂ of patient adapter 28v in comparison with the length L₁ of patient adapter 28u. Furthermore, by virtue of patient adapter 28v it is ensured that, for example, a setting of the depth of focus of Δz = 3 ... 12 mm is possible, the spot diameter of the focal point amounts to 5 µm to 10 µm, and the scan-field diameter amounts to about 7 mm. As a result, a machining of the lens 16b of the eye is made possible despite the use of the same laser device 10.

The above remarks are applicable to the use of the further patient adapters 28w, 28x, 28y, 28z. Also when one of these patient adapters 28w, 28x, 28y, 28z is connected to the same laser device 10, a different treatment region is obtained, for example, through the possibility of a differing setting of the depth of focus and the existence of a differing spot diameter as well as a differing scan-field diameter. A summary of typical values of these is to be found at the end of this description.

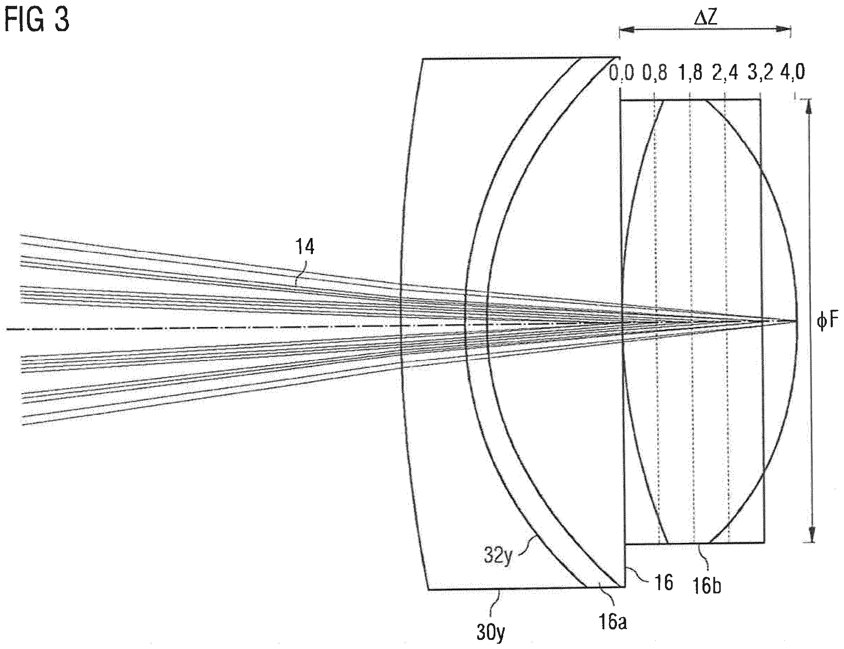

The significance of the aforementioned parameters will be described further on the basis of Figure 3.

In Figure 3 the focus location z₀ of the laser beam 14 may amount in exemplary manner to approximately 0.8 mm. The focus location z₀ specifies how deeply the focal point is situated in the z-direction for a defined state of the z-scan in the eye (here with respect to the anterior surface of the crystalline lens 16b; with respect to abutment face 32y the focus location z₀ amounts in exemplary manner to about 4 mm). The range of depth of focus Δz of the laser beam according to Figure 3 amounts in exemplary manner to about 4 mm and specifies the range of adjustment of the focal point in the z-direction with one and the same laser device 10. The scan-field diameter Φ_{F} of, in exemplary manner, about 8 mm specifies the diameter of the region that is capable of being irradiated in the x-y direction by the laser beam 14 (with one and the same laser device 10). As can be discerned from Figure 3, for the machining of the cornea 16a a larger scan-field diameter Φ_{F} is required than for the machining of the lens 16b. On the other hand, for the machining of the lens 16b higher values for the mean focus location z₀ with respect to abutment face 32y and also a larger range of adjustment Δz are necessary than for the machining of the cornea. However, the values stated for these in exemplary manner are not to be understood as being restrictive but serve merely for illustration.

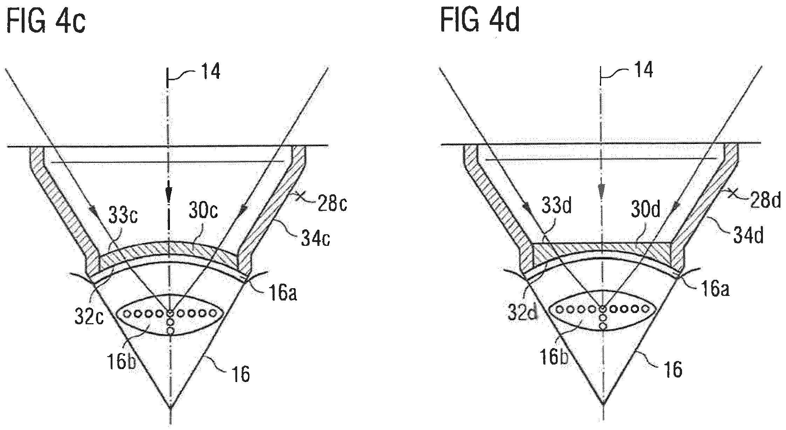

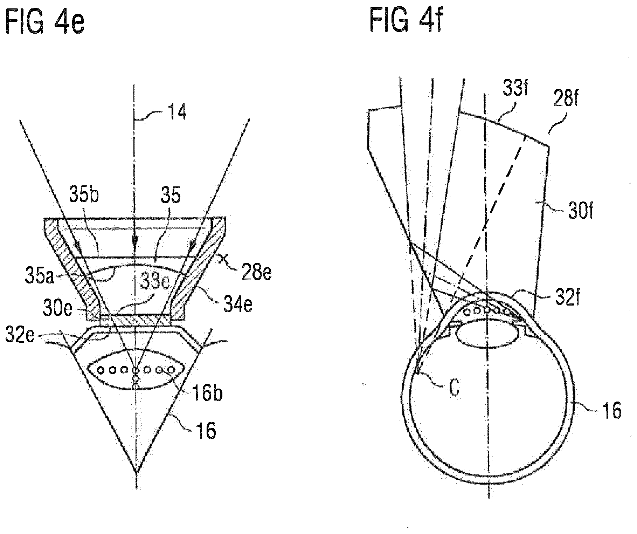

Figures 4a, 4b, 4c, 4d and 4e show various patient adapters 28a, 28b, 28c, 28d and 28e for use with the laser device 10. Depending on the patient adapter 28a, 28b, 28c, 28d and 28e being used, a differing optical effect in the laser device 10 can be brought about. Patient adapter 28a shown in Figure 4a is suitable for implementing treatments of the cornea 16a of the eye 16, such as the implementation of incisions in the cornea 16a, by means of the laser device 10.

Patient adapter 28a is, as shown in Figure 1, detachably coupled with the focusing objective 26 and constitutes an abutment interface for the cornea 16a of the eye 16. For this purpose, patient adapter 28a includes a contact element 30a that is transparent to the laser radiation and that on its underside facing towards the eye includes an abutment face 32a for the cornea 16a. Abutment face 32a is realised in the case of patient adapter 28a as a plane surface and serves for levelling the cornea 16a, by contact element 30a being pressed against the eye 16 with appropriate pressure or by the cornea 16a being aspirated onto abutment face 32a by underpressure. In the case of the plane-parallel design shown in Figure 4a, contact element 30a is ordinarily designated as an applanation plate and is fitted to the narrower end of a conically widening carrier sleeve 34a. The connection between contact element 30a and the carrier sleeve 34a may be permanent, for example by virtue of adhesion bonding, or it may be detachable, for instance by virtue of a screw coupling. Alternatively, an integrally-produced injection-moulded part may find application. In a manner not represented in any detail, the carrier sleeve 34a has at its wider sleeve end, in the drawing the upper end, suitable coupling structures for coupling onto the focusing objective 26.

The laser beam 14, which is indicated schematically in Figure 4a, penetrates the body of the patient adapter 28a, which is transmitting in respect of the laser radiation, and impinges on the planar contact lens 30a. Both faces (the abutment face 32a facing towards the eye 16 and the face 33a facing away from the eye) of the planar contact lens 30a are shaped flat. The eye 16 to be treated bears against the abutment face 32a of the contact lens 30a. After penetrating the contact lens 30a the laser beam 14 impinges on the cornea 16a at a focus indicated schematically. By x-y displacement and z-displacement of the focal point, incisions can now be implemented in the cornea 16a in accordance with the type of incision figure predetermined by the control program.

The reference symbols in Figures 4b to 4e corresponding to the reference symbols from Figure 4a denote the corresponding elements.

Figure 4b shows a patient adapter 28b that is suitable for carrying out treatments in the lens 16b of the eye 16 and capable of being coupled with the focusing objective 26. Just like patient adapter 28a from Figure 4a, patient adapter 28b according to Figure 4b includes a planar contact lens 30b. Patient adapter 28b includes a shorter length L₂ than patient adapter 28a (with a length L₁). That is to say, in comparison with patient adapter 28a according to Figure 4a, which is suitable for the treatment of the cornea 16a, patient adapter 28b according to Figure 4b, which is suitable for the treatment of the lens 16b, is shortened in the z-direction. As can be discerned in Figure 4b, this shortening causes the focal point of the laser beam 14 to come to be situated not in the cornea 16a but in the lens 16b. With the aid of the x-y displacement and also the z-displacement of the focal point, incisions can now be generated in the lens 16b. If the laser beam 14 is deflected laterally, this being indicated on the basis of the further laser beam 14b in Figure 4b, a laterally displaced focal point results in the lens 16b. As indicated schematically in Figure 4b, the focal points have a differing focus diameter, depending upon their focus location in the x-y direction and in the z-direction. As can be discerned in Figure 4b, the focus diameters increase in the lateral direction and in the axial direction, starting from the focal point of the central laser beam 14. This non-uniform focusing in the various depth regions and with lateral beam deflection can be compensated by an increase in the laser-pulse energy, in order to obtain the desired photodisruption threshold also in the marginal regions of the lens 16b. Alternatively, however, the non-uniform focusing may also be compensated by an adaptive optical element, a diffractive optical element or an element shaped with a freeform surface.

Figure 4c shows a patient adapter 28c that is suitable for the treatment of the lens 16b and capable of being coupled with the focusing objective 26. Instead of the planar contact lens 30b used in patient adapter 28b according to Figure 4b, in patient adapter 28c according to Figure 4c use is made of a concavo-convex contact lens 30c. In the case of this concavo-convex contact lens 30c the face 33c facing away from the eye 16 is convexly shaped, whereas the abutment face 32c facing towards the eye 16 (the face bearing against the eye) is concavely shaped. By virtue of the concave shaping of the abutment face 32c facing towards the eye, the rise in intraocular pressure is lessened. The contact lens 30c is shaped in such a manner that the changes in the focus diameter arising in the case of patient adapter 28b from Figure 4b are compensated at the focal points. As can be discerned in Figure 4c, the central focal points (compared with Figure 4b) are either retained (they remain unchanged) or slightly enlarged (worsened), whereas the focus diameters of the focal points in the marginal regions (compared with Figure 4b) both in the lateral direction and in the axial direction are reduced (improved). Therefore the focus diameters of the focal point include an at least almost constant focus diameter, irrespective of the location of the focal point in the lateral and axial directions. The at least almost constant focus diameter may, for example, be obtained by virtue of freeform surfaces formed on the contact lens 30c. For example, the abutment face 32c facing towards the eye and/or the face 33c of the contact lens 30c facing away from the eye may have been shaped as a freeform surface. As a result, the energy of the laser radiation 14 that is necessary for photodisruption in the marginal regions does not have to be increased or increased so intensely as in the case where use is made of patient adapter 28b according to Figure 4b but can be kept at least almost constant.

Patient adapter 28d in Figure 4d differs from patient adapter 28c from Figure 4c only by virtue of the fact that instead of the concavo-convex contact lens 30c use is made of a concavo-planar contact lens 30d. In the case of this concavo-planar contact lens 30d the abutment face 32d facing towards the eye 16 is concavely shaped and the face 33d facing away from the eye is planar. Instead of the concavo-planar contact lens 30d, use may also be made of a concavo-concave contact lens, wherein both the abutment face facing towards the eye 16 and the face facing away from the eye 16 are concavely shaped. The contact lens 32d may also include freeform surfaces on one or both of faces 32d, 33d. As can be discerned in Figure 4d, patient adapter 28d also causes the focus diameters to be at least almost constant both in the lateral direction and in the axial direction.

Patient adapter 28e shown in Figure 4e includes a planar contact lens 30e, wherein both the abutment face 32e (face 32e facing towards the eye) and the face 33e situated opposite the abutment face (face 33e facing away from the eye) are shaped in planar manner. In addition, in patient adapter 28e an optical ancillary element 35 is formed. The optical ancillary element includes a concave face 35a facing towards the eye and a planar face 35b facing away from the eye. One or both of faces 35a, 35b may have been shaped as freeform surfaces. As can be discerned in Figure 4e, the optical ancillary element brings about a diminution of the focus diameters in the marginal regions. In the central regions an enlargement of the focus diameters and hence an adaptation of the focus diameters at all positions in the lens 16b can be brought about. In the central regions the focus diameter can also remain unchanged.

Patient adapter 28f shown in Figure 4f includes a concavo-convex contact lens 30f, wherein the abutment face 32f (face 32f facing towards the eye) is concavely shaped and the face 33f situated opposite the abutment face (face 33f facing away from the eye) is convexly shaped. The contact lens 30f may also include optical freeform surfaces on one or on both of faces 32f, 33f. As can be discerned in Figure 4f, patient adapter 28f also causes the focus diameters to be at least almost constant both in the lateral direction and in the axial direction. Patient adapter 28f from Figure 4f corresponds to patient adapter 28x from Figure 2d.

Irrespective of the element (optical ancillary element 35, contact lens 30c, contact lens 30d) on which one or more freeform surfaces have been formed, the at least one freeform surface may have been matched to an average human eye or may have been formed in patient-individual manner. So a patient adapter may include one or more freeform surfaces which in an average human eye bring(s) about the desired adaptation of the focus diameter. However, it is also conceivable to survey the eye prior to the machining of the human eye and to derive patient-individual data therefrom. From the patient-individual (eye-specific) data, freeform surfaces can be calculated which are then formed in the associated patient-individual patient adapters. As a result, the precision of the machining can be increased. It is similarly conceivable to add wavefront corrections by virtue of the adaptive system taking the form, in exemplary manner, of a mirror 42, in order to increase the precision of the machining.

Furthermore, each of the freeform surfaces may have been provided with an optical coating, in order to reduce reflection losses of the laser radiation 14.

As described in connection with the Figures, with the aid of the differing patient adapters 28a to 28e differing treatments can be carried out with the same laser device 10 even if the settings of the laser device remain unchanged. Consequently a system is made available with which differing types of treatment can be realised with one and the same laser device.
In conclusion a Table, to be regarded as exemplary, will be given which specifies values that are typical (but not to be understood as restrictive) for the purpose of treating a certain region of the eye.

| Treatment region | Mean depth of focus z₀ [mm] starting from corneal surface z = 0 mm | Range of depth of focus Δz [mm] | Necessary focus size ΦF [µm] | Lateral (x-y) scan range [mm] | Necessary laser energy [µJ] |
|---|---|---|---|---|---|
| Cornea | 0.3 | 0.0 ... 1.2 | 3 ... 5 | 12 | 0.5 ... 2.0 |
| Lens | 5.0 | 3.0 ... 10.0 | ≤ 10 | 7 | 2.0 ... 10.0 |
| Vitreous body | 15 | 7 ... ∼ 20 | ≤ 10 | ≥ 15 | 5 ... 10 |
| Retina | 23 | 20 ... 28 | ≤ 5 ... 10 | ≥ 15 | < 1 |
| Iridocorneal angle | 3 | 2 ... 6 | < 10 | ∼ 5 | 10 |

## Claims

1. A set of interface devices (28e) for use in an eye-surgical laser apparatus (10), each of the interface devices including a contact body that is transparent to the laser radiation (14) of the laser apparatus, with an abutment face (32e) configured to abut against an eye (19) to be treated, and also a coupling portion configured to detachably couple of the interface device onto a counter-coupling portion of the laser apparatus,
the interface devices differing by virtue of:
a different influence on the location of a radiation focus of the laser radiation relative to the abutment face and/or
a different shape and/or location of at least one optical boundary surface and/or
a different number of optical elements;
wherein, depending upon the interface device of the set, a different range of depth of focus of the laser radiation in at least one of the x-direction, the y-direction, and the z-direction is obtainable without changing a setting of focusing optics of the laser apparatus,
wherein at least one of the interface devices includes a planar contact lens (30e) with a planar abutment face (32e) configured to abut against the eye and a face (33e) situated opposite the abutment face is adapted to be plane-parallel to the abutment face, and
wherein at least one of the interface devices includes an optical ancillary element (35) arranged in the interface device in such a manner that a face (35b) facing away from the contact lens is shaped in convex or planar manner and a face (35a) facing towards the contact lens is concavely shaped.

2. Set of interface devices according to Claim 1, wherein the face facing towards the contact lens and/or the face facing away from the contact lens is/are formed as a freeform surface.

3. Laser system for eye surgery, comprising:
an eye-surgical laser apparatus (10) comprising:
optical components (22, 24, 26) configured to provide pulsed focused laser radiation with radiation properties matched to the generation of photodisruptions in human eye tissue, the optical components including focusing optics (26), wherein a setting of the focusing optics defines a range of depth of focus of the laser radiation in at least one of the x-direction, the y-direction, and the z-direction; and
a control unit (36) configured to position-control of the radiation focus of the laser radiation, the control unit being configured to execute various control programs (40) that represent various types of incision figures; and
a set of interface devices (28e) according to claim 1.

4. System according to Claim 3, wherein:
at least one of the interface devices includes an applanation cone that is designed to be coupled onto the eye and onto the focusing optics of the laser apparatus.

5. System according to Claim 3 or 4, wherein the laser apparatus further includes an adaptive optical element (42) that is arranged upstream of the focusing optics of the laser apparatus in the direction of propagation of the laser radiation.

6. System according to Claim 5, wherein the adaptive optical element comprises an adaptive mirror (42) or a light-transmitting adaptive system.

7. System according to one any one of claims 3 to 6, wherein at least one of the interface devices includes a coding/code and the laser apparatus is configured to recognise the coding/code and to call an associated control program in the control unit.

## Patentansprüche

1. Satz von Schnittstellenvorrichtungen (28e) zur Verwendung in einem Laserapparat (10) zur Augenchirurgie, wobei jede der Schnittstellenvorrichtungen einen Kontaktkörper einschließt, der für die Laserstrahlung (14) des Laserapparats transparent ist, mit einer Anlagefläche (32e), die zum Anliegen an einem zu behandelnden Auge (19) ausgestaltet ist, und auch mit einem Kopplungsabschnitt, der zum lösbaren Koppeln der Schnittstellenvorrichtung an einen Gegenkopplungsabschnitt des Laserapparats ausgestaltet ist,
wobei die Schnittstellenvorrichtungen sich durch Folgendes unterscheiden:
einen unterschiedlichen Einfluss auf die Position eines Strahlungsfokus der Laserstrahlung relativ zu der Anlagefläche, und/oder
eine unterschiedliche Form und/oder Position von mindestens einer optischen Grenzoberfläche, und/oder
eine unterschiedliche Anzahl an optischen Elementen;
wobei in Abhängigkeit von der Schnittstellenvorrichtung des Satzes ein unterschiedlicher Bereich von Fokustiefe der Laserstrahlung in mindestens einer von der x-Richtung, der y-Richtung und der z-Richtung erhältlich ist, ohne eine Einstellung der Fokussierungsoptik des Laserapparats zu verändern,
wobei mindestens eine der Schnittstellenvorrichtungen eine planare Kontaktlinse (30e) mit einer planaren Anlagefläche (32e) einschließt, die zum Anliegen an dem Auge ausgestaltet ist, und wobei eine Fläche (33e), die sich gegenüber der Anlagefläche befindet, so vorgesehen ist, dass sie ebenenparallel zu der Anlagefläche ist, und
wobei mindestens eine der Schnittstellenvorrichtungen ein optisches Hilfselement (35) einschließt, das in der Schnittstellenvorrichtung in einer solchen Weise angeordnet ist, dass eine Fläche (35b), die von der Kontaktlinse weg weist, in konvexer oder planar Weise geformt ist, und eine Fläche (35a), die zu der Kontaktlinse hin weist, konkav geformt ist.

2. Satz von Schnittstellenvorrichtungen nach Anspruch 1, wobei die Fläche, die zu der Kontaktlinse hin weist, und/oder die Fläche, die von der Kontaktlinse weg weist, als Freiformoberfläche gebildet ist bzw. sind.

3. Lasersystem zur Augenchirurgie, umfassend:
einen Laserapparat (10) für die Augenchirurgie, umfassend:
optische Komponenten (22, 24, 26), die ausgestaltet sind, um gepulste fokussierte Laserstrahlung mit Strahlungseigenschaften bereitzustellen, die mit der Erzeugung von Fotodisruptionen in Gewebe des menschlichen Auges übereinstimmen, wobei die optischen Komponenten Fokussierungsoptik (26) einschließen, wobei eine Einstellung der Fokussierungsoptik einen Bereich der Fokustiefe der Laserstrahlung in mindestens einer von der x-Richtung, der y-Richtung und der z-Richtung definiert; und
eine Steuereinheit (36), die zur Positionssteuerung des Strahlungsfokus der Laserstrahlung ausgestaltet ist, wobei die Steuereinheit ausgestaltet ist, um verschiedene Steuerprogramme (40) auszuführen, die verschiedene Typen von Inzisionsfiguren repräsentieren; und
einen Satz von Schnittstellenvorrichtungen (28e) nach Anspruch 1.

4. System nach Anspruch 3, wobei:
mindestens eine der Schnittstellenvorrichtungen einen Applanationskonus einschließt, der konzipiert ist, um auf das Auge und auf die Fokussierungsoptik des Laserapparats gekoppelt zu werden.

5. System nach Anspruch 3 oder 4, wobei der Laserapparat des Weiteren ein adaptives optisches Element (42) einschließt, das vorgeordnet zu der Fokussierungsoptik des Laserapparats in der Ausbreitungsrichtung der Laserstrahlung angeordnet ist.

6. System nach Anspruch 5, wobei das adaptive optische Element einen adaptiven Spiegel (42) oder ein lichtdurchlässiges adaptives System umfasst.

7. System nach einem der Ansprüche 3 bis 6, wobei mindestens eine der Schnittstellenvorrichtungen eine Codierung/einen Code einschließt, und der Laserapparat konfiguriert ist, um die Codierung/den Code zu erkennen und ein zugehöriges Steuerprogramm in der Steuereinheit aufzurufen.

## Revendications

1. Ensemble de dispositifs d'interface (28e) destinés à être utilisés dans un appareil laser de chirurgie oculaire (10), chacun des dispositifs d'interface comprenant un corps de contact qui est transparent au rayonnement laser (14) de l'appareil laser, comportant une face de butée (32e) configurée pour venir en butée contre un œil (19) à traiter, ainsi qu'une partie de couplage pour un couplage détachable du dispositif d'interface sur une partie de contre-couplage de l'appareil laser,
les dispositifs d'interface étant différents en vertu de :
une influence différente sur l'emplacement d'un foyer de rayonnement du rayonnement laser par rapport à la face de butée et/ou
une forme différente et/ou un emplacement différent d'au moins une surface de limite optique et/ou
un nombre différent d'éléments optiques ;
dans lequel, en fonction du dispositif d'interface de l'ensemble, une plage de profondeur de foyer différente du rayonnement laser dans la direction x et/ou la direction y et/ou la direction z peut être obtenue sans changer un réglage d'optiques de focalisation de l'appareil laser,
dans lequel au moins un des dispositifs d'interface comprend une lentille de contact plane (30e) dotée d'une face de butée (32e) plane configurée pour venir en butée contre l'œil et une face (33e) située en regard de la face de butée est conçue pour être parallèle dans le plan à la face de butée, et
dans lequel au moins un des dispositifs d'interface comprend un élément optique accessoire (35) agencé dans le dispositif d'interface de sorte qu'une face (35b) à l'opposé de la lentille de contact soit de forme convexe ou plane et qu'une face (35a) tournée vers la lentille de contact soit de forme concave.

2. Ensemble de dispositifs d'interface selon la revendication 1, dans lequel la face tournée vers la lentille de contact et/ou la face à l'opposé de la lentille de contact est/sont en forme de surface libre.

3. Système laser pour chirurgie oculaire, comprenant :
un appareil laser pour chirurgie oculaire (10) comprenant :
des composants optiques (22, 24, 26) pour fournir un rayonnement laser focalisé pulsé ayant des propriétés de rayonnement correspondant à la génération de photodisruptions dans un tissu oculaire humain, les composants optiques comprenant des optiques de focalisation (26), dans lequel un réglage des optiques de focalisation définit une plage de profondeur de foyer du rayonnement laser dans la direction x et/ou la direction y et/ou la direction z ; et
une unité de commande (36) configurée pour commander la position du foyer de rayonnement du rayonnement laser, l'unité de commande étant configurée pour exécuter divers programmes de commande (40) qui représentent divers types de figures d'incision ; et
un ensemble de dispositifs d'interface (28e) selon la revendication 1.

4. Système selon la revendication 3, dans lequel :
au moins un des dispositifs d'interface comprend un cône d'aplanation qui est conçu pour être accouplé sur l'œil et sur les optiques de focalisation de l'appareil laser.

5. Système selon la revendication 3 ou 4, dans lequel l'appareil laser comprend en outre un élément optique adaptatif (42) qui est agencé en amont des optiques de focalisation de l'appareil laser dans le sens de propagation du rayonnement laser.

6. Système selon la revendication 5, dans lequel l'élément optique adaptatif comprend un miroir adaptatif (42) ou un système adaptatif de transmission lumineuse.

7. Système selon l'une quelconque des revendications 3 à 6, dans lequel au moins un des dispositifs d'interface comprend un codage/code et l'appareil laser est conçu pour reconnaître le codage/code et pour appeler un programme de commande associé dans l'unité de commande.
